# FASCICULE DE BREVET EUROPEEN

(11) **EP 1 549 280 B1**
(45) Date de publication et mention de la délivrance du brevet: **22.06.2011**
(21) Numéro de dépôt: 03797346.8
(22) Date de dépôt: 18.09.2003
(51) Int. Cl.: A61Q 1/10, A61K 8/892, A61K 8/895

(54) **COMPOSITION DE MAQUILLAGE , EN PARTICULIER DES CILS**
SCHMINKE, INSBESONDERE FÜR WIMPERN
MAKE-UP, PARTICULARLY FOR EYESLASHES

(30) Priorité: 19.09.2002 FR 0211588
(43) Date de publication de la demande: 06.07.2005
(73) Titulaire: LVMH RECHERCHE, 45800 St. Jean de Braye (FR)
(72) Inventeur: GOUAISBAULT, Rosemary, F-45000 Orléans (FR); FAURE-TROMEUR, Mélanie, F-45520 Cercottes (FR); KUENTZ-MURA, Annie, F-45450 Faye aux Loges (FR); TRANCHANT, Jean-François, F-45760 Marigny les Usages (FR)
(74) Mandataire: Chantraine, Sylvie Hélène
(86) Numéro de dépôt international: PCT/FR2003/002747
(87) Numéro de publication internationale: WO 2004/026221

(56) Documents cités:
- EP-A- 0 862 913
- EP-A- 0 953 332
- EP-A- 0 979 642
- US-B1- 6 406 683

## Description

La présente invention concerne le domaine du maquillage des fibres kératiniques, en particulier des cils.

Plus précisément, l'invention concerne un nouveau procédé de maquillage des fibres kératiniques, selon lequel on applique sur ces fibres, une composition permettant de former sur ces fibres, des gouttes de préférence transparentes, brillantes, qui sèchent rapidement sans coller les unes aux autres, et persistent dans le temps, ce qui conduit à l'obtention d'un effet purement esthétique lié à la formation de ces gouttes transparentes.

L'invention concerne également de nouvelles compositions permettant d'obtenir cette formation de gouttes de préférence transparentes lors de leur application sur les cils ou les cheveux.

La demande de brevet EP 0 953 332 décrit une composition pour le maquillage des fibres kératiniques, notamment des cils et des cheveux, contenant une dispersion aqueuse de polymère et son utilisation pour le maquillage des fibres kératiniques. Cette composition, à condition d'être appliquée dans des conditions particulières, permet de réaliser un dépôt discontinu sur les fibres kératiniques sensiblement longitudinales, sous forme de gouttelettes successives. Les polymères utilisés pour la réalisation de ces compositions ont des viscosités qui sont typiquement de l'ordre de celles des milieux aqueux, ce qui explique qu'il est nécessaire, pour obtenir une viscosité suffisante, d'ajouter des agents viscosifiants dans les compositions pour obtenir la formation de gouttes sur la fibre kératinique.

Les inventeurs de la présente invention ont maintenant découvert qu'il était possible de réaliser des compositions permettant de déposer des gouttes sur les fibres kératiniques, en particulier sur les cils et les cheveux, sans avoir besoin de recourir dans la composition à des agents viscosifiants.

Plus précisément, cette invention résulte de recherches des inventeurs sur des polymères aux propriétés rhéologiques particulières, et en particulier sur des polymères siliconés.

Il est bien connu que les problèmes liés à la mouillabilité des substrats sont particulièrement complexes, tant d'un point de vue théorique que pratique et l'étude des propriétés de l'interface liquide/solide fait l'objet de nombreuses recherches fondamentales et appliquées dont les retombées industrielles et économiques sont nombreuses.

Or, aucune étude à ce jour ne vise à élucider le problème consistant à former des gouttes ou des gouttelettes ayant les propriétés précitées sur des fibres kératiniques.

Cette aptitude à réaliser une telle formation de gouttes dépend non seulement de la nature du substrat mais également de la nature de la composition à déposer, et de la quantité déposée.

Les inventeurs de la présente invention ont établi qu'il était possible d'atteindre l'effet recherché en utilisant une composition contenant, à titre de constituants essentiels, un solvant volatil et un polymère aux propriétés rhéologiques (viscoélasticité, viscosité) particulières.

Ainsi, selon un premier aspect, l'invention concerne un nouveau procédé de maquillage utilisant de telles compositions pour former des gouttes sur des fibres kératiniques, en particulier sur les cils et les cheveux.

D'autre part, l'invention concerne des compositions nouvelles utilisables dans cette application, en particulier des compositions liquides à la température ambiante.

Plus précisément, l'invention concerne un procédé de maquillage des fibres kératiniques en particulier des cils ou des cheveux, destiné à former des gouttes sur ces fibres, caractérisé en ce qu'il comprend l'application sur lesdites fibres d'une composition contenant de 5 à 30% en poids d'un diméthiconol linéaire et présentant:
- une viscoélasticité caractérisée par un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
   une viscosité dynamique de l'ordre de 6400 Pa.s à 25°C ; dispersé dans un solvant volatil,
   ladite composition ne contenant pas de produit à effet modificateur de viscoélasticité, susceptible d'empêcher la formation desdites gouttes à la concentration utilisée.
   Selon un autre mode de réalisation de l'invention, l'invention 20 concerne aussi un procédé de maquillage des fibres kératiniques en particulier des cils ou des cheveux, destiné à former des gouttes sur ces fibres, caractérisé en ce qu'il comprend l'application sur lesdites fibres d'une composition consistant essentiellement en, ou consistant en,
   une dispersion dans un solvant volatil de 5 à 30% en poids par rapport au poids de ladite composition d'un diméthiconol linéaire
   présentant :
- une viscoélasticité caractérisée par un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
- une viscosité dynamique de l'ordre de 6400 Pa.s à 25°C

Ainsi, l'invention résulte essentiellement de la corrélation établie par les inventeurs entre les propriétés rhéologiques des polymères et la possibilité d'obtenir des gouttes sur les fibres kératiniques par application sur ces fibres d'une solution de ces polymères dans un solvant volatil.

Toutes les mesures de viscoélasticité et de viscosité dynamiques données dans le présent document sont effectuées à une température de l'ordre de 25°C, avec un appareil Rheometric Dynamic Analyser RDAII, commercialisé par la société Rheometrics, USA, et en suivant la procédure et la notice d'utilisation du constructeur. Comme cela est connu, les mesures de viscosité sont des mesures non destructurantes réalisées dans un plateau newtonien où il est bien connu que l'on peut fixer alternativement la valeur de la fréquence ou celle de la contrainte appliquée au système.

Le rhéomètre utilisé pour réaliser les mesures dans le cadre de la présente invention présente une géométrie de plaques parallèles. Les mesures nécessaires sont ensuite faites à des déformations constantes ou fréquences constantes.

On notera que les caractéristiques de viscoélasticité du polymère sont :
- le module G' de conservation qui caractérise l'élasticité,
- le module G " de perte qui caractérise la viscosité.

Les polymères retenus sont ceux pour lesquels les courbes représentant les modules G' et G" en fonction de la fréquence présentent un point d'intersection dans l'intervalle compris entre 0.3 Hz et 3 Hz, de préférence entre 0,5 et 1,5 Hz, de préférence encore au voisinage de 1 Hz.

En effet, pour des fréquences inférieures à l'intervalle retenu, il apparaît que ces polymères sont dans un état trop liquide et par conséquent s'écoulent, les chaînes polymériques ayant tendance à se désenchevêtrer.

En revanche, au-delà de cet intervalle, ces polymères passent par un état trop caoutchoutique.

On notera également que les courbes de viscosité traduisent des mesures faites en régime dynamique et représentent en abscisses la déformation et en ordonnées, la viscosité dynamique.

Tous les polymères répondant aux caractéristiques rhéologiques exposées ci-dessus peuvent être utilisés pour la préparation des compositions utiles selon l'invention.

A titre d'exemple, on choisira de préférence le polymère diméthiconol commercialisé par Dow Corning, notamment sous le nom de SGM-36®, ayant une viscosité de 6400 Pa.s à 25°C.

Les solvants volatils utilisés pour la préparation des compositions utilisées selon l'invention peuvent être avantageusement tous les solvants volatils habituellement utilisés en cosmétique dans la mesure où ils dispersent le polymère choisi et sont compatibles avec la formation de gouttes.

A titre de solvant volatil, on choisira de préférence un produit qui s'évapore sur la peau à température ambiante.

Ce solvant volatil aura de préférence une pression de vapeur saturante à pression atmosphérique et température ambiante, inférieure à 3 Pa.

On choisira de préférence un solvant volatil de type silicone linéaire ou cyclique, tel que les diméthicones linéaires possédant de 2 à 9 atomes de silicium, les cyclométhicones possédant de 3 à 8 atomes de silicium.

Le solvant volatil préféré est l'hexaméthyldisiloxane qui permet l'obtention de gouttes transparentes, brillantes, qui sèchent rapidement sans coller les unes aux autres et persistent dans le temps.

A titre d'exemple, on choisira de préférence le solvant volatil commercialisé par Dow Corning sous le nom de Dow Corning 200 0.65 cts.

Ainsi, selon un mode de réalisation de l'invention, on peut fabriquer des compositions anhydres.

Les proportions de diméthiconol linéaire et de solvant volatil contenus dans les compositions utilisées selon l'invention peuvent varier dans de larges gammes. Toutefois, la concentration diméthiconol linéaire est de 5 à 30 %, et de préférence de 10 à 25 % en poids par rapport au poids de la composition de maquillage. De préférence, la concentration de polymère(s) est de 15 à 25 % en poids par rapport au poids de la composition de maquillage.

Comme exposé précédemment, une des caractéristiques recherchées pour les compositions utilisées selon l'invention est de conduire, lors de leur application sur les fibres kératiniques, à des gouttes ou gouttelettes qui ne collent pas entre elles.

Toutefois, il peut être avantageux d'introduire dans la composition un produit destiné à diminuer le caractère collant des gouttes.

Pour remplir cette fonction, on peut choisir avantageusement un produit habituellement utilisé pour améliorer le caractère « sans transfert » des compositions cosmétiques.

Un exemple de produit particulièrement avantageux à cet effet est constitué d'un mélange d'un polymère de diméthicone réticulé par la vinyldiméthicone, dans un solvant constitué par la cyclométhicone D5, ayant un cycle à 5 silicium, ledit produit se trouvant de préférence à une concentration comprise entre 5 et 15% en poids par rapport au poids de la composition.

Un exemple d'un tel produit est le produit commercialisé par Shin-Etsu Silicones Europe B.V sous le nom commercial KSG-15® qui est un mélange de polymères réticulés contenu dans des proportions de 2 à 10 % de polymère de diméthicone réticulé et 90 à 98 % de cyclométhicone D5.

Ainsi, les compositions cosmétiques ou de maquillage obtenues présentent un remarquable effet esthétique à la lumière du fait du dépôt sur les cils de gouttes et de préférence présentant un caractère transparent.

Selon une autre variante, les compositions de l'invention peuvent être également appliquées sur les cheveux avec le même objectif de fixer des gouttes.

Selon encore une variante de l'invention, on pourra introduire dans la composition un additif cosmétiquement acceptable non modificateur de viscoélasticité à la concentration utilisée, tel que agent colorant, agent parfumant, agent conservateur, agent antioxydant , filtre UV, etc.

Selon une variante de l'invention, il pourrait être recherché la formation de gouttes transparentes et colorées.

Cette coloration pourra être obtenue par l'introduction dans les compositions utilisées d'un colorant organique soluble et/ou de charges minérales pulvérulentes, avantageusement multicouches ou composites à effets visuels telles que des nacres.

Cette coloration des gouttes permettra d'obtenir des effets de lumière particuliers, contribuant au caractère esthétique des gouttes.

Selon un deuxième aspect, la présente invention concerne aussi une composition notamment destinée à maquiller les fibres kératiniques, en particulier les cils ou les cheveux, en formant des gouttes à leurs extrémités lors de son application, comprenant au moins un diméthiconol linéaire présentant les caractéristiques de viscoélasticité et de viscosité dynamique telles que précédemment définies dans le cadre du procédé et comme définies dans les revendications indépendantes de composition, ladite composition ne contenant pas de produit à effet modificateur de viscoélasticité, susceptible d'empêcher la formation desdites gouttes, à la concentration utilisée et ledit polymère étant dispersé dans un solvant volatil.

Selon un autre mode de réalisation de l'invention, l'invention concerne aussi une composition notamment destinée à maquiller les fibres kératiniques, en particulier les cils ou les cheveux, en formant des gouttes à leurs extrémités lors de son application, consistant essentiellement en, diméthiconol linéaire ou consistant en, ledit
et présentant des caractéristiques de viscoélasticité et de viscosité dynamique telles que définies pour la composition précédente ou dans le cadre du procédé de maquillage et telles que définies dans la revendication de composition indépendante, ledit polymère ou mélange de polymères étant dispersé dans un solvant volatil.

Les caractéristiques avantageuses de chacune de ces compositions selon l'invention font l'objet des sous-revendications de composition, qui sont incorporées dans leur totalité par référence, ces variantes de réalisation étant également décrites dans le cadre du procédé de maquillage, et s'appliquant naturellement à ce deuxième aspect de l'invention visant les compositions.

Selon une variante particulièrement avantageuse, la composition de l'invention contient un diméthiconol linéaire ayant une viscosité d'environ 6400 Pa.s à 25°C en solution dans un solvant volatil comprenant l'hexaméthyldisiloxane.

Les exemples qui suivent sont donnés à titre purement illustratif de l'invention. Dans les exemples, tous les pourcentages sont donnés en poids, la température est la température ambiante ou est exprimée en degré Celsius, la pression est la pression atmosphérique, sauf indications contraires.

### PRESENTATION DES FIGURES

Les exemples sont complétés par les figures 1 à 6 qui représentent respectivement :
Figure 1 : les paramètres de viscoélasticité d'un polymère diméthiconol utilisable pour la mise en oeuvre de l'invention (commercialisé par Dow Corning sous la dénomination SGM36®),
Figure 2 : la courbe de viscosité du même polymère,
Figure 3 : une photographie de goutte obtenue dans l'étude goniométrique de la composition contenant le polymère diméthiconol, pour un temps de contact de 15 secondes, sur un cil orienté ver le haut,
Figure 4 : une photographie de la même goutte qu'à la figure 3 obtenue dans l'étude goniométrique, mais pour un temps de contact de 90 secondes,
Figure 5 : une photographie de la même goutte que celle de la figure 3 ou 4, mais pour un temps de contact de 300 secondes, et
Figure 6 : une goutte formée sur un cil saturé de ladite composition et orienté vers le bas après un temps de contact de 45 minutes.

### EXEMPLES

### I. CARACTERISATIONS RHEOLOGIQUES DES POLYMERES

Les caractérisations de la viscoélasticité et de la viscosité des polymères, ont été réalisées à 25°C et sous pression atmosphérique sur le polymère pur, à l'aide d'un rhéomètre de type Rheometric Dynamic Analyser RDAII, commercialisé par la société Rheometrics, USA, dans les conditions suivantes préconisées par la notice d'utilisation du constructeur.

### 1. Viscoélasticité

Les modules G' et G" sont suivis en fonction de la fréquence appliquée au moyen dudit rhéomètre de type Rheometric Dynamic Analyser RDAII.

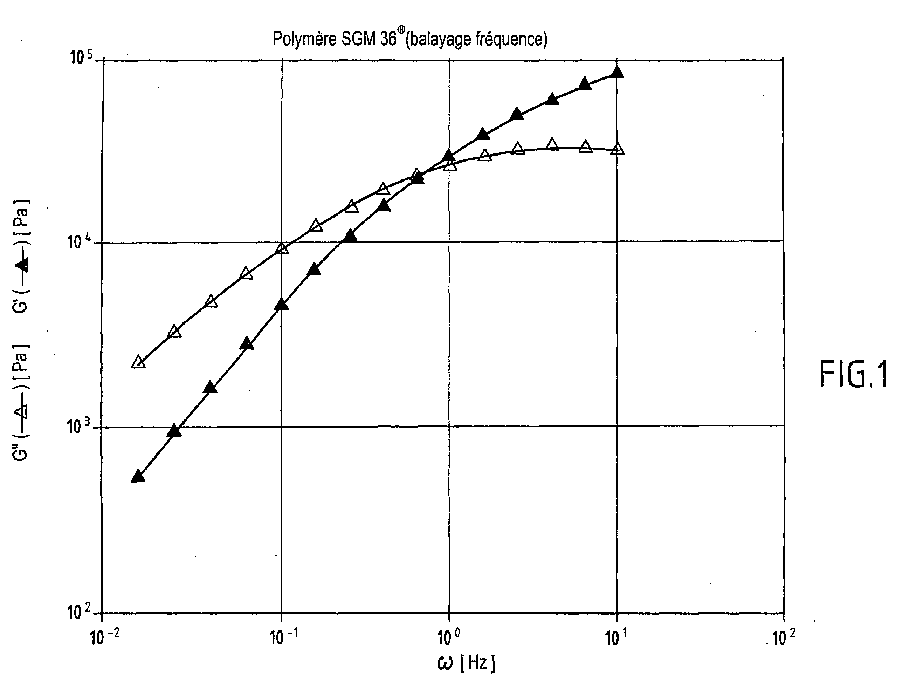

La figure 1 ci-jointe est un exemple de courbes représentatives de l'évolution de G' et G" avec la fréquence ω̅ pour le polymère diméthiconol SGM36® de Dow Corning dont le point d'intersection des courbes représentant G' et G" correspond à une fréquence de l'ordre de 1 Hz.

### 2. Viscosité

La viscosité a été étudiée à l'aide dudit rhéomètre.

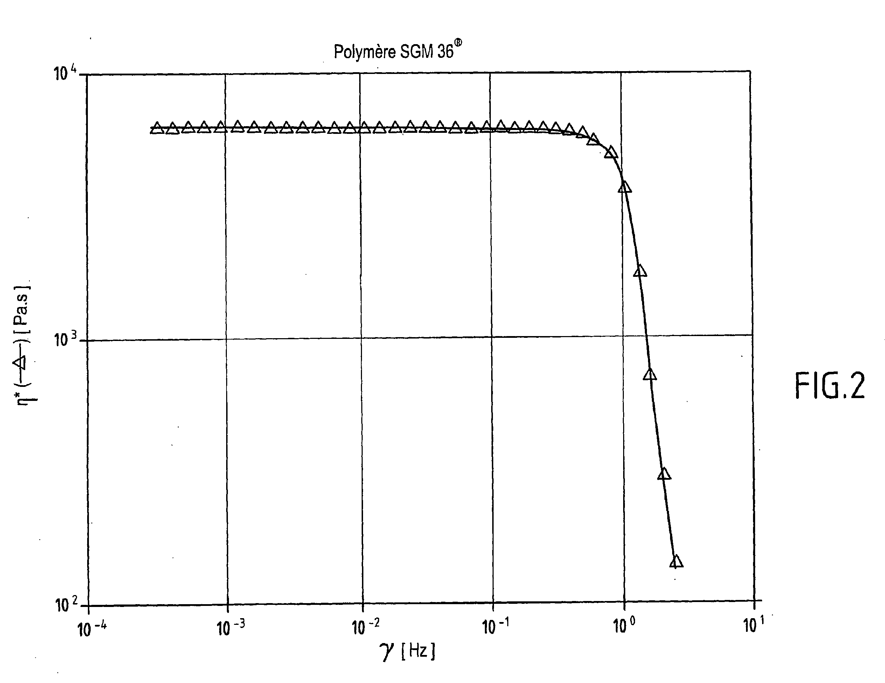

Un exemple de courbe de viscosité du polymère convenant pour l'invention, ici à savoir le polymère diméthiconol référence SGM36® de Dow Corning, est donné à la figure 2.

### II. PREPARATION ET EVALUATION DE COMPOSITIONS UTILISABLES SELON L'INVENTION

### 1. Préparation

Les différentes compositions sont toutes réalisées avec un homogénéisateur de type Rayneri.

On dissout 19,2 parties en poids de polymère diméthiconol SGM36® dans 72,8 parties en poids de solvant héxaméthyldisiloxane de chez Dow Corning, référence Dow Corning 200 0.65 cts, à l'aide de l'homogénéisateur puis on ajoute 8 parties en poids d'un additif antitransfert diminuant le pouvoir collant, ici un mélange de cyclométhicone D5 et d'un polymère de diméthicone réticulé dans le vinyldiméthicone, commercialisé sous la marque KSG15® précitée, et l'agitation est maintenue jusqu'à l'obtention d'un liquide visqueux homogène et transparent.

Le produit ainsi obtenu est prêt à l'application.

### 2. Application

On distinguera dans l'exemple 2 modes d'application possibles :

### 2.1. Application avec une tige filetée

1. Le produit à tester est contenu dans un flacon en verre de 30 ml, référence 7409001263 de VG emballages.
2. On dispose des faux-cils en cheveux naturels, de marque L.J.C. référence 63-200, sur une colonne en plexiglass.
3. On se munit d'une tige filetée référence 151 Silver Cap 500NS-140-3.250 de Henlopen.
4. On plonge cette tige (partie filetée) dans le produit à tester, et on ôte l'excédent de produit sur le rebord du flacon ; la tige est ainsi chargée de produit en quantité suffisante pour en déposer sur les cils, et juste assez pour ne pas couler de la tige avant application ; on estime à environ 0.15 g la quantité nécessaire de produit pour charger convenablement la tige filetée utilisée.
5. Ensuite on brosse les cils de la base vers le bout, une fois au dessus, puis une fois en dessous.
6. On charge à nouveau la tige comme en [4] puis on vient tapoter le bout des cils pour définitivement positionner les gouttes. La dimension optimale de celles-ci est d'environ 1 à 2 mm.
7. Le séchage s'opère en quelques minutes de façon naturelle à l'air libre.

### 2.2 Application avec un pinceau

Cette variante, qui consiste à prélever du produit avec la pointe d'un pinceau (type pinceau à lèvres) et à déposer ce produit juste au bout des cils, permet de placer précisément les gouttes et d'en moduler la taille.

### 3. Evaluation

Outre l'appréciation visuelle de la formation des gouttes, on a réalisé également un suivi par goniométrie de la formation des gouttes dans les conditions suivantes :

L'application est réalisée à l'aide d'une brosse mascara standard

Le liquide, est déposé sur le cil sec ou préalablement saturé, à son extrémité libre, avec la solution à tester.

Le dépôt est réalisé soit sur cil orienté vers le bas soit sur cil orienté vers le haut.

L'acquisition des images est effectuée en mode statique à l'aide d'une caméra numérique équipée d'un zoom 18-108/2,5 fonctionnant en mode macro. La mise au point se fait dans le plan de la fibre kératinique à une distance zoom/cil d'environ 160 mm avec un rétro-éclairage par rampe de diodes de couleurs rouges.

Plus précisément, cette acquisition d'images est réalisée, dans un premier temps, en mode cinétique, afin d'observer l'évolution aux temps courts (juste après le dépôt du liquide sur le cil), à raison d'une image toutes les 15 secondes, puis en mode statique dans le but d'observer l'évolution de la forme des gouttes aux temps longs avec prise d'images ponctuelle.

Le traitement des images et la détermination de l'angle de contact sont réalisés en recourant aux logiciels de type WinCalc^{®} et WinGoutte^{®} et au moyen d'un goniomètre classique du commerce permettant de mesurer l'angle de contact entre la goutte et la surface du cil.

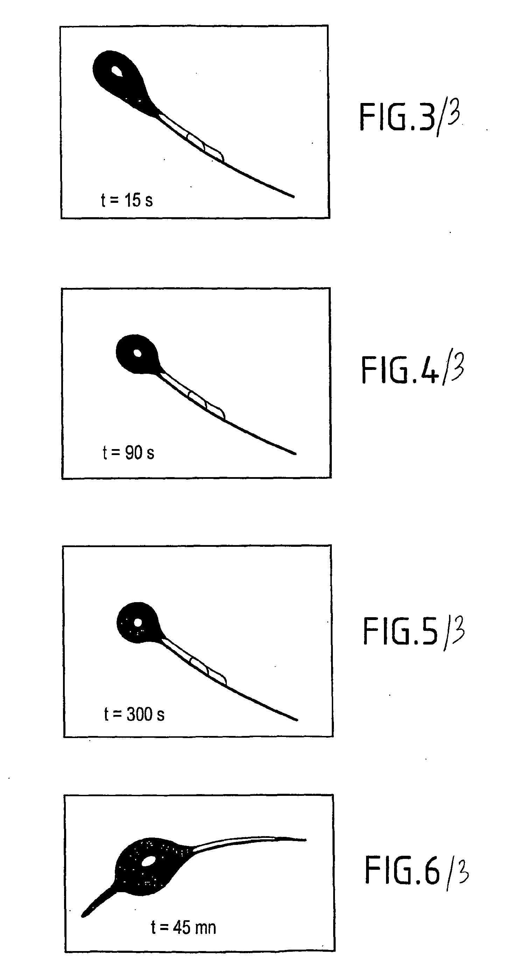

Un exemple d'aspect de gouttes est donné sur la figure 3, pour un dépôt à l'extrémité d'un cil sec orienté vers le haut, après 15 secondes ;

la figure 4 représente l'aspect de la goutte similaire à la figure 3, mais pour un temps de séjour de 90 secondes, ce qui permet d'observer déjà la formation de la goutte de manière plus circulaire ;

la figure 5 représente l'aspect de la goutte après 300 secondes, ce qui permet d'observer la formation rapide d'une goutte sphérique, avec peu d'évolution au temps long, donc une très bonne stabilité ; et

la figure 6 représente un autre exemple d'aspect de goutte sur un cil orienté vers le bas, saturé avec la même composition, pour un temps de contact de 45 minutes, où l'on peut encore constater une excellente stabilité de la goutte au cours du temps.

Ainsi, la composition selon l'invention permet de former des gouttes relativement sphériques, stables dans le temps, et qui sont de préférence transparentes.

## Revendications

1. Procédé de maquillage des fibres kératiniques, en particulier des cils ou des cheveux, destiné à former des gouttes sur ces fibres, **caractérisé en ce qu'**il comprend l'application sur lesdites fibres d'une composition contenant de 5 à 30% en poids d'un diméthiconol linéaire présentant :
- une viscoélasticité **caractérisée par** un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
- une viscosité dynamique de l'ordre de 6 400 Pa.s à 2.5°C ;
et dispersé dans un solvant volatil,
ladite composition ne contenant pas de produit à effet modificateur de viscoélasticité, susceptible d'empêcher la formation desdites gouttes, à la concentration utilisée.

2. Procédé de maquillage des fibres kératiniques, en particulier des cils ou des cheveux, destiné à former des gouttes sur ces fibres, **caractérisé en ce qu'**il comprend l'application sur les fibres d'une composition constituée essentiellement de, ou constituée de, une dispersion dans un solvant volatil de 5 à 30% en poids par rapport au poids de ladite composition d'un diméthiconol linéaire présentant :
- une viscoélasticité **caractérisée par** un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
- une viscosité dynamique de l'ordre de 6 400 Pa.s à 25°C.

3. Procédé selon l'une des revendications 1 ou 2, **caractérisé en ce que** ledit solvant volatil est choisi parmi une diméthicone linéaire possédant de 2 à 9 atomes de silicium, une cyclométhicone possédant de 3 à 8 atomes de silicium.

4. Procédé selon l'une des revendications 1 à 3, **caractérisé en ce que** ledit solvant volatil est l'hexaméthyldisiloxane.

5. Procédé selon l'une des revendications 1 à 4, **caractérisé en ce que** la concentration en diméthiconol est comprise entre 10 et 25% en poids par rapport au poids de la composition de maquillage.

6. Procédé selon la revendication précédente 5, **caractérisé en ce que** la concentration en diméthiconol est de 15 à 25% en poids par rapport au poids de la composition de maquillage.

7. Procédé selon l'une des revendications 1 ou 3 à 6, **caractérisé en ce que** ladite composition contient en outre un produit destiné à diminuer le caractère collant des gouttes.

8. Procédé selon la revendication 7, **caractérisé en ce que** ledit produit est un mélange d'une cyclométhicone D5 et d'un polymère de diméthicone réticulé par la vinyldiméthicone, de préférence à une concentration comprise entre 5 et 15% en poids par rapport au poids de la composition.

9. Procédé selon l'une des revendications 1 à 8, **caractérisé en ce que** lesdites fibres kératiniques sont les cils.

10. Procédé selon l'une des revendications 1 à 8, **caractérisé en ce que** lesdites fibres kératiniques sont les cheveux.

11. Procédé selon l'une des revendications 1 ou 3 à 10, **caractérisé en ce que** la composition contient un additif cosmétiquement acceptable non modificateur de viscoélasticité à la concentration utilisée, choisi dans le groupe des agents colorants, des agents parfumants, des agents conservateurs, des agents antioxydants, et des filtres UV.

12. Composition notamment destinée à maquiller les fibres kératiniques, en particulier les cils ou les cheveux, en formant des gouttes à leurs extrémités lors de son application, comprenant 5 à 30% en poids d'un diméthiconol linéaire présentant :
- une viscoélasticité **caractérisée par** un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
- une viscosité dynamique de l'ordre de 6 400 Pa.s à 25°C,
ledit diméthiconol étant dispersé dans un solvant volatil,
ladite composition ne contenant pas de produit à effet modificateur de viscoélasticité, susceptible d'empêcher la formation desdites gouttes, à la concentration utilisée.

13. Composition notamment destinée à maquiller les fibres kératiniques, en particulier les cils ou les cheveux, en formant des gouttes à leurs extrémités lors de son application, constituée essentiellement de, ou constituée de, une dispersion dans un solvant volatil de 5 à 30% en poids par rapport au poids de ladite composition d'un diméthiconol linéaire présentant :
- une viscoélasticité **caractérisée par** un module de conservation G' et un module de perte G", tels que G' soit inférieur à G" pour des fréquences inférieures à 0,3 Hz et supérieur à G" pour des fréquences supérieures à 3 Hz, les deux courbes représentatives de G' et G" en fonction de la fréquence présentant un point d'intersection dans l'intervalle compris entre 0,3 et 3 Hz, de préférence entre 0,5 et 1,5 Hz, de préférence encore au voisinage de 1 Hz, et
- une viscosité dynamique de l'ordre de 6 400 Pa.s à 25°C.

14. Composition selon l'une des revendications 12 ou 13, **caractérisée en ce que** ledit solvant volatil, est choisi parmi une diméthicone linéaire possédant de 2 à 9 atomes de silicium, une cyclométhicone possédant de 3 à 8 atomes de silicium.

15. Composition selon l'une des revendications 12 à 14, **caractérisée en ce que** ledit solvant volatil est l'hexaméthyldisiloxane.

16. Composition selon l'une des revendications 12 à 15, **caractérisée en ce que** la concentration en diméthiconol est comprise entre 10 et 25% en poids par rapport au poids de la composition de maquillage.

17. Composition selon la revendication 16, **caractérisée en ce que** la concentration en diméthiconol est de 15 à 25% en poids par rapport au poids de la composition de maquillage

18. Composition selon l'une des revendications 12 ou 14 à 17, **caractérisée en ce que** ladite composition contient en outre un produit destiné à diminuer le caractère collant des gouttes.

19. Composition selon la revendication 18, **caractérisée en ce que** ledit produit est un mélange d'une cyclométhicone D5 et d'un polymère de diméthicone réticulé par la vinyldiméthicone, de préférence à une concentration comprise entre 5 et 15% en poids par rapport au poids de la composition.

20. Composition selon la revendication 19, **caractérisée en ce que** la composition contient un additif cosmétiquement acceptable non modificateur de viscoélasticité à la concentration utilisée, choisi dans le groupe des agents colorants, des agents parfumants, des agents conservateurs, des agents antioxydants, et des filtres UV.

## Claims

1. A method of make-up of keratin fibres, particularly of eyelashes or the hair, intended to form drops on these fibres, **characterised in that** it comprises applying, onto said fibres, a composition containing from 5% to 30% by weight of a linear dimethiconol having:
- a viscoelasticity **characterised by** a conservation modulus G' and a loss modulus G", which are such that G' be less than G" for frequencies of lower than 0.3 Hz and greater than G" for frequencies of higher than 3 Hz, the two curves representing G' and G" as a function of the frequency having a point of intersection in the interval comprised between 0.3 and 3 Hz, preferably between 0.5 and 1.5 Hz, more preferably of about 1Hz, and
- a dynamic viscosity of about 6,400 Pa.s at 25°C;
dispersed in a volatile solvent,
said composition containing no product having a viscoelasticity-modifying effect, which can prevent the formation of said drops, at the concentration used.

2. A method of make-up of keratin fibres, particularly of eyelashes or the hair, intended to form drops on these fibres, **characterised in that** it comprises applying, onto said fibres, a composition which essentially consists of, or which consists of, a dispersion in a volatile solvent from 5% to 30% by weight with respect to the weight of said composition of a linear dimethiconol which has:
- a viscoelasticity **characterised by** a conservation modulus G' and a loss modulus G", which are such that G' be less than G" for frequencies of lower than 0.3 Hz and greater than G" for frequencies of higher than 3 Hz, the two curves representing G' and G" as a function of the frequency having a point of intersection in the interval comprised between 0.3 and 3 Hz, preferably between 0.5 and 1.5 Hz, more preferably of about 1 Hz,
and
- a dynamic viscosity of about 6,400 Pa.s at 25°C.

3. The method according to claim 1 or 2, **characterised in that** said volatile solvent is selected from a linear dimethicone having 2 to 9 silicon atoms, and a cyclomethicone having 3 to 8 silicon atoms.

4. The method according to one of claims 1 to 3, **characterised in that** said volatile solvent is hexamethyldisiloxane.

5. The method according to one of claims 1 to 4, **characterised in that** the concentration of dimethyconol is between 10% and 25% by weight with respect to the weight of the make-up composition.

6. The method according to claim 5, **characterised in that** the concentration of dimethiconol is 15 to 25% by weight with respect to the weight of the make-up composition.

7. The method according to one of claims 1 or 3 to 6, **characterised in that** said composition further contains a product intended to reduce the sticky character of the drops.

8. The method according to claim 7, **characterised in that** said product is a mixture of a cyclomethicone D5 and a dimethicone polymer which is cross-linked by vinyldimethicone, preferably at a concentration comprised between 5 and 15% by weight with respect to the weight of the composition.

9. The method according to one of claims 1 to 8, **characterised in that** said keratin fibres are eyelashes.

10. The method according to one of claims 1 to 8, **characterised in that** said keratin fibres are the hair.

11. The method according to one of claims 1 or 3 to 10, **characterised in that** the composition contains a cosmetically-acceptable additive which is non-viscoelasticity-modifying at the concentration used, chosen from the group consisting of colouring agents, perfuming agents, preserving agents, anti-oxidizing agents, and UV-filters.

12. A composition notably intended for the make-up of keratin fibres, particularly eyelashes or the hair, in forming drops at their tips upon its application, comprising from 5 to 30% by weight of a linear dimethiconol which has:
- a viscoelasticity **characterised by** a conservation modulus G' and a loss modulus G", which are such that G' be less than G" for frequencies of lower than 0.3 Hz and greater than G" for frequencies of higher than 3 Hz, the two curves representing G' and G" as a function of the frequency having a point of intersection in the interval comprised between 0.3 and 3 Hz, preferably between 0.5 and 1.5 Hz, more preferably of about 1 Hz, and
- a dynamic viscosity of about 6,400 Pa.s at 25°C,
said dimethiconol being dispersed in a volatile solvent,
said composition containing no product having a viscoelasticity-modifying effect, which can prevent the formation of said drops, at the concentration used.

13. A composition notably intended for the make-up of keratin fibres, particularly eyelashes or the hair, in forming drops at their tips upon its application, and which essentially consists of, or which consists of, a dispersion in a volatile solvent from 5 to 30% by weight with respect to the weight of said composition of a linear dimethiconol which has :
- a viscoelasticity **characterised by** a conservation modulus G' and a loss modulus G", which are such that G' be less than G" for frequencies of lower than 0.3 Hz and greater than G" for frequencies of higher than 3 Hz, the two curves representing G' and G" as a function of the frequency having a point of intersection in the interval comprised between 0.3 and 3 Hz, preferably between 0.5 and 1.5 Hz, more preferably of about 1 Hz, and
- a dynamic viscosity of about 6,400 Pa.s at 25°C,

14. The composition according to claim 12 or 13, **characterised in that** said volatile solvent is selected from a linear dimethicone having 2 to 9 silicon atoms, and a cyclomethicone having 3 to 8 silicon atoms.

15. The composition according to one of claims 12 to 14, **characterised in that** said volatile solvent is hexamethyldisiloxane.

16. The composition according to one of claims 12 to 15, **characterised in that** the concentration of dimethiconol is comprised between 10 and 25% by weight with respect to the weight of the make-up composition.

17. The composition according to claim 16, **characterised in that** the concentration of dimethiconol is from 15 to 25% by weight with respect to the weight of the make-up composition.

18. The composition according to one of claims 12 or 14 to 17, **characterised in that** said composition further contains a product intended to reduce the sticky character of the drops.

19. The composition according to claim 18, **characterised in that** said product is a mixture of a cyclomethicone D5 and a dimethicone polymer which is cross-linked by vinyldimethicone, preferably at a concentration comprised between 5 and 15% by weight with respect to the weight of the composition.

20. The composition according to claim 19, **characterised in that** the composition contains a cosmetically-acceptable additive which is non-viscoelasticity-modifying at the concentration used, chosen from the group consisting of colouring agents, perfuming agents, preserving agents, anti-oxidizing agents, and UV-filters.

## Patentansprüche

1. Verfahren zum Schminken von Keratinfasern, insbesondere Wimpern oder Haaren, das dazu bestimmt ist, auf diesen Fasern Tropfen zu erzeugen, **dadurch gekennzeichnet, daß** es das Auftragen einer Zusammensetzung auf die Fasern umfaßt,
die 5 bis 30 Gew.-% eines linearen Dimethiconols enthält und aufweist:
- eine Viskoelastizität, **gekennzeichnet durch** ein Konservierungsmodul G' und ein Verlustmodul G", so daß G' kleiner als G" für Frequenzen unter 0,3 Hz und größer als G" für Frequenzen über 3 Hz ist, wobei die beiden repräsentativen Kurven G' und G" in Abhängigkeit von der Frequenz einen Schnittpunkt in dem Intervall zwischen 0,3 und 3 Hz, vorzugsweise zwischen 0,5 und 1,5 Hz, ferner vorzugsweise in der Nähe von 1 Hz aufweisen, und
- eine dynamische Viskosität von ungefähr 6400 Pa.s bei 25 °C,
und die in einem flüchtigen Lösungsmittel aufgelöst wird,
wobei die Zusammensetzung kein Produkt mit die Viskoelastizität verändernder Wirkung enthält, das die Bildung dieser Tropfen bei der verwendeten Konzentration verhindern kann.

2. Verfahren zum Schminken von Keratinfasern, insbesondere Wimpern oder Haaren, das dazu bestimmt ist, auf diesen Fasern Tropfen zu erzeugen, **dadurch gekennzeichnet, daß** es das Auftragen einer Zusammensetzung auf die Fasern umfaßt, die im wesentlichen oder ganz gebildet wird von einer Dispersion in einem flüchtigen Lösungsmittel von 5 bis 30 Gew.-% bezogen auf das Gewicht der Zusammensetzung eines linearen Dimethiconols, die Folgendes aufweist:
- eine Viskoelastizität, **gekennzeichnet durch** ein Konservierungsmodul G' und ein Verlustmodul G", so daß G' kleiner als G" für Frequenzen unter 0,3 Hz und größer als G" für Frequenzen über 3 Hz ist, wobei die beiden repräsentativen Kurven G' und G" in Abhängigkeit von der Frequenz einen Schnittpunkt in dem Intervall zwischen 0,3 und 3 Hz, vorzugsweise zwischen 0,5 und 1,5 Hz, ferner vorzugsweise in der Nähe von 1 Hz aufweisen, und
- eine dynamische Viskosität von ungefähr 6400 Pa.s bei 25 °C.

3. Verfahren nach einem der Ansprüche 1 oder 2, **dadurch gekennzeichnet, daß** das flüchtige Lösungsmittel unter einem linearen Dimethicon, das 2 bis 9 Siliziumatome besitzt, und einem Cyclomethicon, das 3 bis 8 Siliziumatome besitzt, ausgewählt wird.

4. Verfahren nach einem der Ansprüche 1 bis 3, **dadurch gekennzeichnet, daß** das flüchtige Lösungsmittel Hexamethyldisiloxan ist.

5. Verfahren nach einem der Ansprüche 1 bis 4, **dadurch gekennzeichnet, daß** die Konzentration an Dimethiconol zwischen 10 und 25 Gew.-% bezogen auf das Gewicht der Schminkzusammensetzung beträgt.

6. Verfahren nach dem vorhergehenden Anspruch 5, **dadurch gekennzeichnet, daß** die Konzentration an Dimethiconol 15 bis 25 Gew.-% bezogen auf das Gewicht der Schminkzusammensetzung beträgt.

7. Verfahren nach einem der Ansprüche 1 oder 3 bis 6, **dadurch gekennzeichnet, daß** die Zusammensetzung ferner ein Produkt enthält, das dazu bestimmt ist, die Klebeeigenschaft der Tropfen zu verringern.

8. Verfahren nach Anspruch 7, **dadurch gekennzeichnet, daß** das Produkt ein Gemisch eines Cyclomethicons D5 und eines Dimethiconpolymers, das mit Vinyldimethicon vernetzt ist, ist, vorzugsweise in einer Konzentration zwischen 5 und 15 Gew.-% bezogen auf das Gewicht der Zusammensetzung.

9. Verfahren nach einem der Ansprüche 1 bis 8, **dadurch gekennzeichnet, daß** die Keratinfasern die Wimpern sind.

10. Verfahren nach einem der Ansprüche 1 bis 8, **dadurch gekennzeichnet, daß** die Keratinfasern die Haare sind.

11. Verfahren nach einem der Ansprüche 1 oder 3 bis 10, **dadurch gekennzeichnet, daß** die Zusammensetzung einen kosmetisch annehmbaren Zusatzstoff enthält, der die Viskoelastizität bei der verwendeten Konzentration nicht verändert und aus der Gruppe der Farbstoffe, Duftstoffe, Konservierungsstoffe, Antioxidationsmittel und UV-Filter ausgewählt wird.

12. Zusammensetzung, die insbesondere dazu bestimmt ist, die Keratinfasern, insbesondere die Wimpern oder die Haare, zu schminken, wobei bei ihrem Auftragen an ihren Enden Tropfen gebildet werden, umfassend 5 bis 30 Gew.-% eines linearen Dimethiconols, das aufweist:
- eine Viskoelastizität, **gekennzeichnet durch** ein Konservierungsmodul G' und ein Verlustmodul G", so daß G' kleiner als G" für Frequenzen unter 0,3 Hz und größer als G" für Frequenzen über 3 Hz ist, wobei die beiden repräsentativen Kurven G' und G" in Abhängigkeit von der Frequenz einen Schnittpunkt in dem Intervall zwischen 0,3 und 3 Hz, vorzugsweise zwischen 0,5 und 1,5 Hz, ferner vorzugsweise in der Nähe von 1 Hz aufweisen, und
- eine dynamische Viskosität von ungefähr 6400 Pa.s bei 25 °C,
wobei das Dimethiconol in einem flüchtigen Lösungsmittel aufgelöst wird und
wobei die Zusammensetzung kein Produkt mit die Viskoelastizität verändernder Wirkung enthält, das die Bildung dieser Tropfen bei der verwendeten Konzentration verhindern kann.

13. Zusammensetzung, die insbesondere dazu bestimmt ist, die Keratinfasern, insbesondere die Wimpern oder die Haare, zu schminken und an deren ihren Enden beim Auftragen Tropfen zu bilden und die im wesentlichen oder ganz gebildet ist von einer Dispersion in einem flüchtigen Lösungsmittel von 5 bis 30 Gew.-% bezogen auf das Gewicht der Zusammensetzung eines linearen Dimethiconols, die Folgendes aufweist:
- eine Viskoelastizität, **gekennzeichnet durch** ein Konservierungsmodul G' und ein Verlustmodul G", so daß G' kleiner als G" für Frequenzen unter 0,3 Hz und größer als G" für Frequenzen über 3 Hz ist, wobei die beiden repräsentativen Kurven G' und G" in Abhängigkeit von der Frequenz einen Schnittpunkt in dem Intervall zwischen 0,3 und 3 Hz, vorzugsweise zwischen 0,5 und 1,5 Hz, ferner vorzugsweise in der Nähe von 1 Hz aufweisen, und
- eine dynamische Viskosität von ungefähr 6400 Pa.s bei 25 °C.

14. Zusammensetzung nach einem der Ansprüche 12 oder 13, **dadurch gekennzeichnet, daß** das flüchtige Lösungsmittel unter einem linearen Dimethicon, das 2 bis 9 Siliziumatome besitzt, und einem Cyclomethicon, das 3 bis 8 Siliziumatome besitzt, ausgewählt wird.

15. Zusammensetzung nach einem der Ansprüche 12 bis 14, **dadurch gekennzeichnet, daß** das flüchtige Lösungsmittel Hexamethyldisiloxan ist.

16. Zusammensetzung nach einem der Ansprüche 12 bis 15, **dadurch gekennzeichnet, daß** die Konzentration an Dimethiconol zwischen 10 und 25 Gew.-% bezogen auf das Gewicht der Schminkzusammensetzung beträgt.

17. Zusammensetzung nach Anspruch 16, **dadurch gekennzeichnet, daß** die Konzentration an Dimethiconol 15 bis 25 Gew.-% bezogen auf das Gewicht der Schminkzusammensetzung beträgt.

18. Zusammensetzung nach einem der Ansprüche 12 oder 14 bis 17, **dadurch gekennzeichnet, daß** die Zusammensetzung ferner ein Produkt enthält, das dazu bestimmt ist, die Klebeeigenschaft der Tropfen zu verringern.

19. Zusammensetzung nach Anspruch 18, **dadurch gekennzeichnet, daß** das Produkt ein Gemisch eines Cyclomethicons D5 und eines Dimethiconpolymers, das mit Vinyldimethicon vernetzt ist, ist, vorzugsweise in einer Konzentration zwischen 5 und 15 Gew.-% bezogen auf das Gewicht der Zusammensetzung.

20. Zusammensetzung nach Anspruch 19, **dadurch gekennzeichnet, daß** die Zusammensetzung einen kosmetisch annehmbaren Zusatzstoff enthält, der die Viskoelastizität bei der verwendeten Konzentration nicht verändert und aus der Gruppe der Farbstoffe, Duftstoffe, Konservierungsstoffe, Antioxidationsmittel und UV-Filter ausgewählt wird.
